# EUROPEAN PATENT APPLICATION

(11) **EP 2 060 914 A2**
(43) Date of publication of application: **20.05.2009**
(21) Application number: 09154167.2
(22) Date of filing: 15.03.2006
(51) Int. Cl.: G01N 33/50, C12Q 1/00

(54) **Nutritional compositions for modulating vitamin C bio-availability**

(30) Priority: 15.03.2005 EP 05005569
(62) Divisional of application: 06707563.0
(71) Applicant: Nestec S.A., 1800 Vevey (CH)
(72) Inventor: Steiling, Heike, 1820, Montreux (CH); Williamson, Gary, Harrogate, HG2 7AX (GB); Richelle, Myriam, 1073, Savigny (CH)
(74) Representative: Chautard, Cécile

(57) **Abstract**

The present invention relates to compositions able to modulate Vitamin C bio-availability in individuals. In particular, the present invention pertains to methods for identifying compounds, capable of modulating expression of Vitamin C transporters, the use of said compounds in foods or foodstuffs and the use thereof for the treatment or prevention of diseased or stressed tissue.

## Description

The present invention relates to nutritional and pharmaceutical compositions modulating Vitamin C bio-availability in individuals. In particular, the present invention pertains to methods for identifying compounds, capable of modulating expression of Vitamin C transporters, the use of said compounds in nutritional and pharmaceutical compositions and the use thereof for the treatment or prevention of diseased or stressed tissue.

Vitamins are acknowledged to play diverse roles in the metabolic function of the body.

For example the fat-soluble Vitamin A (retinal), is known to be metabolized to yield various derivatives called retinoids, which are useful in the treatment or prevention of night blindness. Also, retinoids or Vitamin A, respectively, are used as therapy to improve the appearance of sun-damaged skin.

Another Vitamin of great interest is Vitamin C (ascorbic acid, 2,3-didehydro-L-threo-hexano-1,4-lactone), which seems to be a multifactorial component in the biological processes occurring in the body.

Vitamin C is required for many enzymatic reactions involving the biosynthesis of connective-tissue components, including elastin, fibronectin, proteoglycans, bone matrix and elastin-associated fibrillin. Moreover, it appears to play a role in collagen gene expression and cellular procollagen secretion. Ascorbic acid is also a cofactor in the carnitine biosynthetic pathway and is involved in modulating iron absorption, transport and storage. It aids in the intestinal absorption of iron by reducing ferric iron to ferrous iron and may stimulate ferritin synthesis to promote iron storage in cells. Also, it is involved in the biosynthesis of corticosteroids, aldosterone, the conversion of cholesterol to bile acids.

Above all, Vitamin C is known for its antioxidative properties. Studies indicate that a defective serum antioxidant status contributes to the development of a number of diseases, such as coronary heart diseases, cancer, and neurodegenerative diseases, often caused by an oxidative stress in the central nervous system.

Obviously, Vitamin C seems to be effective in preventing the onset of a number of various types of cancer, including bladder, breast, cervical, colorectal, esophageal, lung, pancreatic, prostate, salivary gland, stomach, leukemia, and non-Hodgkin's lymphoma. It is also known that Vitamin C plays an important role in the treatment or prevention of effects brought about by detrimental effects exerted on the skin.

Vitamins are not synthesized in the body, but must be furnished from exogenous sources, generally with the diet. However, western type food is normally characterized to contain too high an amount of fat, while being deficient of Vitamins, so that in most cases the supply of a number of Vitamins is sub-optimal. Under these circumstances, it is common practice to supply Vitamins in form of a supplement.

Such supplements may comprise one Vitamin only or a number thereof, in which case the products are referred to as so-called multi-Vitamin formulations. Also, Vitamins may be supplied to particular areas of the body for the treatment or prevention of a disease, such as e.g. via skin care products.

A problem of all of the present administration forms of Vitamins resides in providing the Vitamin in a bio-available form, i.e. in a form the body/cells may easily absorb. As an example, an individual may be supplied with a given amount of ascorbic acid, produced by e.g. chemical means, yet will absorb a limited amount thereof only, since obviously adjuvant components seem to be needed to maximize this purpose. Specifically, this is of interest in case large doses, i.e. an overdose, of Vitamins are to be employed for the treatment of a disorder, so that such large doses may exert pharmacodynamic actions useful in therapy.

Thus, a problem of the present invention resides in providing novel means for delivering Vitamins to an individual in a bio-available form.

This problem has been solved by providing compounds and/or nutritional and pharmaceutical compositions capable of modulating the transcription of Vitamin C transporters in cells such that the up-take of Vitamin C in the target cells is increased and the up-take of Vitamin C may be actively promoted and controlled, or the up-take of Vitamin C in the target cells is decreased.

In the extensive experimental studies leading to the present invention, the named inventors noted that in a cell culture experiment certain compounds modulated the intracellular level of Vitamin C. Following this first finding, the cells were further investigated while it could be established that for example the lowering of intracellular Vitamin C was accompanied by an lowering in the level of Vitamin C transporter mRNA. Without wishing to be bound by any theory, it is presently believed that such compounds capable of modulating the transcription of Vitamin C transporters in cells act on the gene transcription of Vitamin C transporter genes resulting in an increased or decreased level of said protein with the effect that either Vitamin C up-take by the target cells is augmented or the Vitamin C up-take by the cells is reduced or even disabled, respectively.

Recent studies revealed the existence of two sodium-dependent Vitamin C transporters (SVCT1 and SVCT2, gene symbol: SLC23A1 and SLC23A2) in mammals including man. In situ hybridizations on rat tissues confined SVCT1 expression to epithelial surfaces such as intestine, kidney and liver, whereas SVCT2 is widely expressed including brain, eye adrenal and pituitary glands, lung, stomach and testis. It has been demonstrated that SVCT1 is largely confined to bulk-transporting epithelia, where it serves whole-body homeostasis. So far, the expression of SVCTs in skin has not been reported.

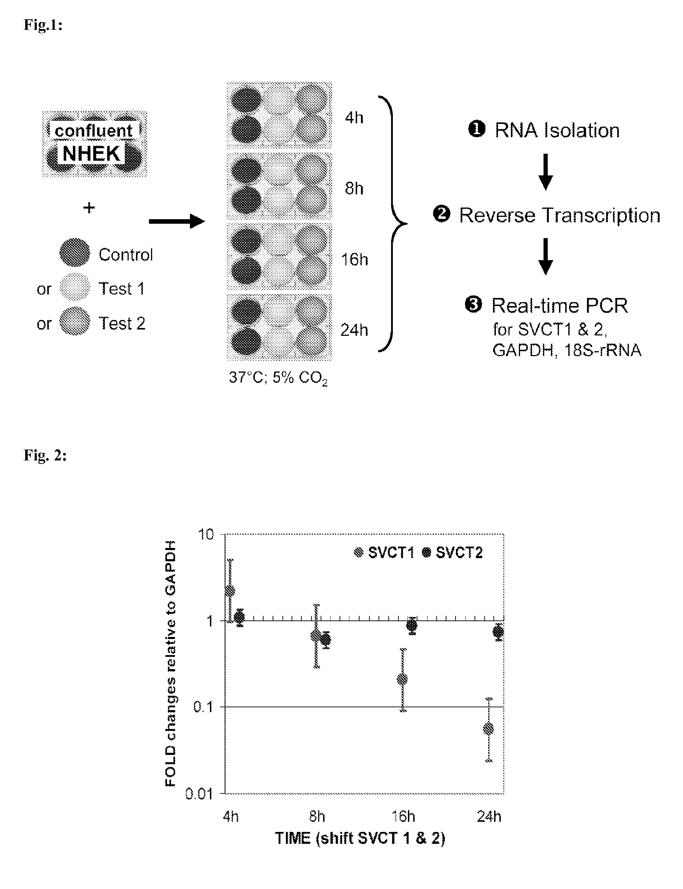

Fig. 1 shows a chart representing the experimental set-up for in vitro test of SVCT gene expression. Confluent primary keratinocytes (NHEK) were treated with a test compound or control, incubated 4h, 8h, 16h or 24h at 37°C. The total cellular RNA was isolated and subsequently used for reverse transcription and real-time PCR analysis of SVCT1 and SVCT2. 18S-rRNA and GAPDH mRNA analysis served as endogenous control (housekeeping genes).

Fig. 2 shows a real-time PCR analysis of primary keratinocytes treated with 2.5% rosemary extract. Fold changes in relative expression of primary keratinocytes (NHEK) treated with rosemary extract compared to control cells and relative to GAPDH are shown. Dots represent averages of 4 biological samples with technical triplicates each. The control cells at 4h were set to 1 fold and is represented by the graded line. Confident intervals were calculated using ANOVA. Data are statistically significant if arrow bars do not touch the 1 fold line.

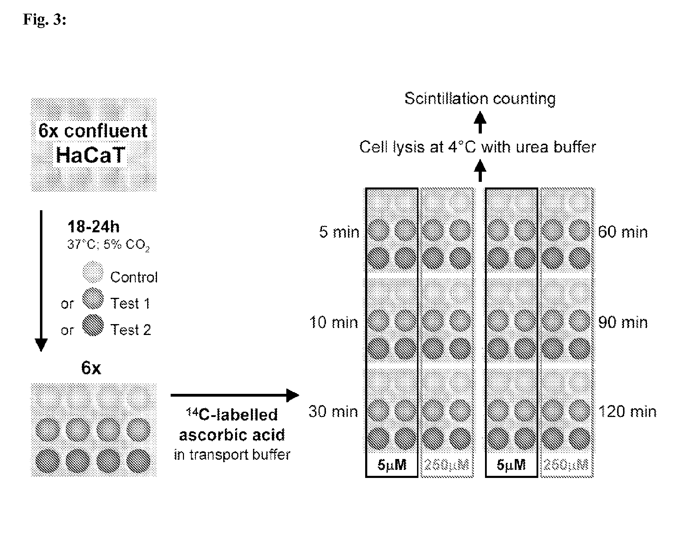

Fig. 3 shows a chart representing the experimental set-up for in vitro test of ascorbic acid up-take. Confluent HaCaT cells were treated for 18-24h with a test compound or control, incubated with ¹⁴C-labelled ascorbic acid at two different concentrations (5 µM and 250 µM) for 5 min, 10 min, 30 min, 60 min, 90 min and 120 min. Cells were lysed and the radiolabel in the supernatant and the cell lysate was quantified by scintillation counting.

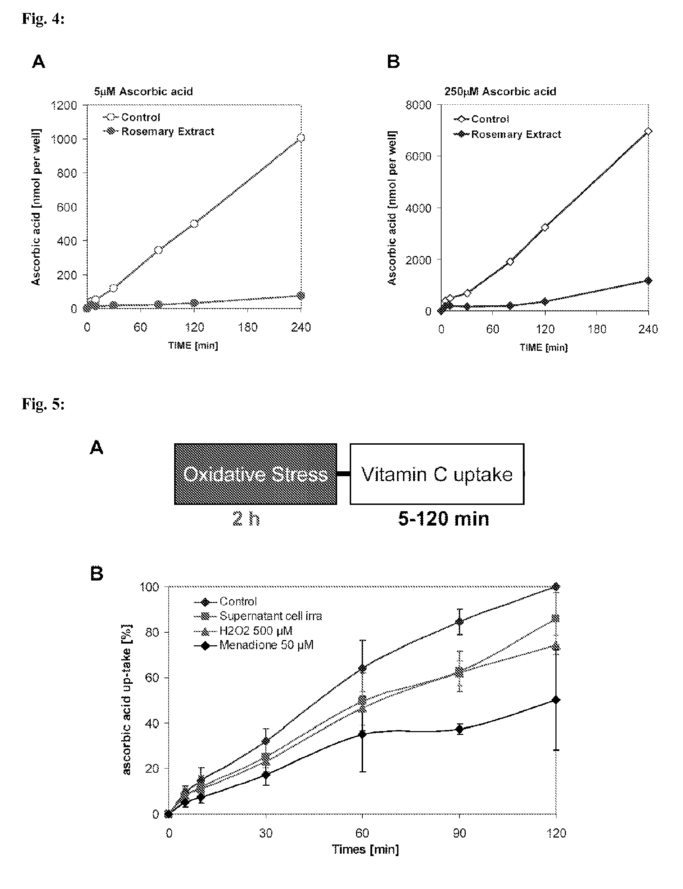

Fig. 4 shows the ascorbic acid up-take in primary keratinocytes treated with and without 2.5% rosemary extract. NHEK were treated for 24h with 2.5% rosemary extract (filled circles) or with control (open circles) and up-take of ¹⁴C-labelled ascorbic acid was measured after different time points and expressed as nmol per well (A: 5 µM ascorbic acid, B: 250 µM ascorbic acid). Data represents averages of two replicates and were re-produced in an independent set of experiments (data not shown).

Fig. 5 shows an in vitro test of ascorbic acid up-take in a model of oxidative stress. Confluent HaCaT cells were treated for 2h with 50 µM menadione (black rhombus), 500 µM H₂O₂ (triangles), conditioned medium obtained from UV-irradiated HaCaT cells (squares) or control (grey rhombus) and subsequently incubated with 50 µM ¹⁴C-labelled ascorbic acid for 5 min, 10 min, 30 min, 60 min, 90 min and 120 min. Cells were lysed and the radiolabel in supernatant and cell lysate quantified by scintillation counting. A chart representing the experimental timelines is shown in A. Data shown in B represent averages with corresponding standard deviations of 3 independent experiments with 2 technical replicates each.

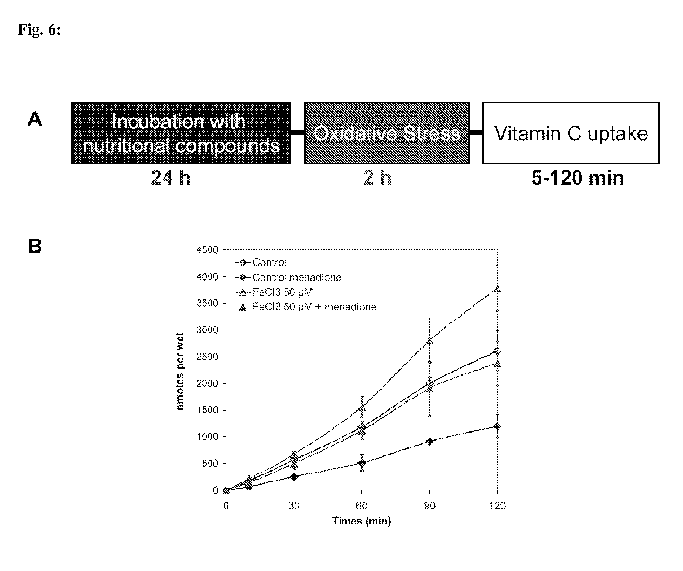

Fig. 6 shows an in-vitro test of ascorbic acid up-take in a model of oxidative stress. Confluent HaCaT cells were treated with 50 µM FeCl₃ (triangles) or controls (grey rhombus) for 24h and for additional 2h with 50 µM menadione (filled symbols) or controls (open symbols). Subsequently, cells were incubated with 50 µM ¹⁴C-labelled ascorbic acid for 5 min, 10 min, 30 min, 60 min, 90 min and 120 min, cells were lysed and the radiolabel in supernatant and cell lysate quantified by scintillation counting. A chart representing the experimental timelines is shown in A. Data shown in B represent averages with corresponding standard deviations of 2 independent experiments with 2 technical replicates each.

According to an embodiment, the present invention provides compounds capable of modulating the intracellular level of Vitamin C. The compounds may be obtained by a method comprising the steps of (a) preparing a cell culture; (b) exposing said cell culture to an appropriate amount of the compound to be identified and an appropriate amount of Vitamin C; (c) determining the level of Vitamin C or of the expression rate of Vitamin C transporters in the tissue cell; and (d) comparing the expression rate of the Vitamin C transporters with a control.

In general, for carrying out the method outlined above, the cell culture may be performed with cells derived from any kind of living being, e.g. animals including humans, either from a cell line or from a tissue to give a primary cell culture. The cells are preferably of mammalian origin, more preferably of human or pet origin. According to an embodiment, the cells are epithelial cells, which are, according to a preferred embodiment, melanocytes and/or keratinocytes, which for the ease of the cell culture may be immortalized.

The cells are grown under conventional conditions e.g. in RPMI medium, KGM medium or Dulbecco's modified Eagle medium to a certain number, which may be determined by a Thoma Chamber or any other appropriate method known to the skilled person.

Subsequently, a test compound is added to the medium and either at the same time or after a given period of time a predetermined amount of Vitamin C. The amount of Vitamin C to be added to the cell culture is preferably in a range of 0.1 µM to 10 mM, more preferably in a range of from 0.1 µM to 0.5 mM, while the amount of the test-compound are in a range of 0.1 µM to 100 µM for pure compounds and 0.001% (w/v) to 0.5% (w/v) for natural-product extracts, more preferably in a range of 1 µM to 100 µM for pure compounds and 0.01% (w/v) to 0.5% (w/v) for natural-product extracts. The preparation of extracts is known to the skilled person. Extracts may be for example any kind of aqueous extracts or extracts with ethyl acetate either hydrolyzed or non-hydrolyzed. The test compound and the Vitamin C may be added as powder or in a dissolved form.

In a next step, the effect of the test substance on the expression of Vitamin C transporters is determined. This may be achieved by either determining the level of Vitamin C transporter mRNA or protein, or the amount of Vitamin C absorbed or remaining in the culture supernatant.

According to a preferred embodiment the effect of the test substance is determined by asserting the transcription of Vitamin C transporters, preferably the Vitamin C transporters SVCT1 and SVCT2 (gene symbol: SLC23A1 and SLC23A2).

After a short incubation period, cells are separated from the medium by centrifugation. The expression of the Vitamin C transporter mRNA is visualised via real-time PCR and/or northern blots and may be also quantified.

In the real-time PCR the initial amount of the template is quantified specifically, sensitively and reproducibly, wherein the amount of the final amplified product at the end-point is detected. Real-time PCR monitors the fluorescence emitted during the reaction as an indicator of amplicon production during each PCR cycle as opposed to the endpoint detection. The real-time progress of the reaction may be viewed in some systems. Real-time PCR does not detect the size of the amplicon, however, it is not influenced by non-specific amplification unless SYBR Green or other suitable fluorescent dyes are used. Real-time PCR quantification eliminates post-PCR processing of PCR products (which is necessary in competitive RT-PCR). This increases throughput and reduces the chances of carryover contamination. In comparison to conventional PCR, real-time PCR also offers a much wider dynamic range. Dynamic range of any assay determines how much target concentration can vary and still be quantified. A Real-time PCR may be performed preferably in 96 well format to achieve a high throughput of the samples.

Northern blots allow the determination the molecular weight of an mRNA, their discrimination and further to measure relative amounts of the mRNA present. Messenger RNA (mRNA) is first separated by gel electrophoresis, usually an agarose gel. Afterwards the RNA is transferred to a sheet of blotting paper, preferably nitrocellulose, though other types of paper, or membranes, can be used. The RNA molecules retain the same pattern of separation they had on the gel. The blot is incubated with a probe, which is single-stranded DNA. This probe will form base pairs with its complementary RNA sequence and bind to form a double-stranded RNA-DNA hybrid. The probe cannot be seen but it is either labelled radioactive or has an enzyme bound to it (e.g. alkaline phosphatase, horseradish peroxidase or a fluorescent enzyme like the green fluorescent protein). Then the location of the probe is revealed by incubating it with a corresponding substrate that the attached enzyme converts to a visible coloured product or gives off light which will expose X-ray film. If the probe was labelled with radioactivity, it can be exposed to the X-ray film directly. Afterwards the bands obtained are compared with control samples from healthy and diseased or stressed tissue cells. In particular, the size and intensity of the bands are of interest, which may be compared visually or quantified via a photo-imaging device.

According to a preferred embodiment said Vitamin C transporter proteins are the human sodium-dependent Vitamin C transporters SVCT 1 and/or SVCT 2.

The increase of transcription of a Vitamin C transporter is indicative of the capacity of the test substance to increase Vitamin C uptake by the cell, whereas the decrease of transcription of a Vitamin C transporter indicates the ability of the test substance to lower Vitamin C uptake by the cell.

Alternatively, the amount of Vitamin C absorbed by the cells may be determined directly, e.g. using ¹⁴C-labeled vitamin C or via the reductive properties of Vitamin C, since cells are capable to store a surplus of said Vitamin for a certain period of time. Cells are broken down by suitable procedures, including mechanical and chemical methods. Subsequently, the detection may be performed by scintillation counting in case of ¹⁴C-labeled vitamin C or the reduction of sodium 2,6-dichlorphenolindopenole (DCPIP, Tillmans Reagent), Fehling's Solution, Benedict Solution, Lugol's Solution or the titration with potassium iodate. The determination via high performance/high pressure liquid chromatograph (HPLC) may comprise UV-VIS detection at a wavelength of 265 nm, fluorescence detection after derivatization of dehydro-ascorbic-acid with o-phenyldiamine to a fluorescent product and electrochemical detection using e.g. graphite electrodes. Free radicals may be also detected directly by electron paramagnetic resonance (EPR). Other methods comprise also, but are not limited thereto, mass spectrometry (MS) or gas chromatography (GC). It will be appreciated that the respective results are compared with a negative control.

The compounds to be investigated comprise any chemical substance, capable of influencing/modulating the expression of a transporter protein. Said compounds may be preferably derived natural-product extracts, phytochemicals, amino acids, fatty acids cholesterol, minerals and sugars.

According to an embodiment rosemary extract from different origins (aqueous origin and ethyl acetate extracts (hydrolyzed and non-hydrolyzed)) modifies the transcription of a Vitamin C transporter in that the transcription of SVCT1 mRNA is decreased and Vitamin C uptake by the cell is lowered. Other compounds capable of down-regulating the transcription of SVCT1 mRNA are some phytochemicals such as sulforaphane, all-trans retinoic acid and zinc or a salt thereof, depending on the respective application a nutritional, pharmaceutical or dermatological acceptable salt such as ZnCl₂, significantly down-regulated SVCT1 mRNA expression. Said compounds exert their effects in a time dependent manner. It has been found that these findings are not due to cytotoxic effects of the test compounds, but indicate real potential in reducing SVCT1 mRNA levels.

According to another embodiment iron or salts thereof, depending on the respective application a nutritional, pharmaceutical or dermatological acceptable salt such as FeCl₃, modify the transcription of a Vitamin C transporter in that its transcription is increased and Vitamin C uptake by the cell is enlarged. Other compounds showing this trait are glutathione, N-acetyl-cysteine and lysine. This indicates that food compounds different origins are also capable to increase ascorbic acid bio-availability for tissues. It has been surprisingly found that the effect of the above-mentioned compounds capable of increasing transcription of Vitamin C transporter may be even intensified/fortified by combining two or more of such compounds, such as FeCl₃ and glutathione.

According to an alternative embodiment the present invention also provides a composition, containing a compound having the above outlined trait in that the transcription of a Vitamin C transporter is either increased or decreased, and optionally Vitamin C.

In principle, the compound may be included in any nutritional composition, such as beverages, e.g. cold or warm drinks, such as soft drinks or tea, or to foodstuff, e.g. to sauces, included in spices, etc.. For example, in some embodiments, the compound may be provided in or mixed with a beverage, or stirred into a semi-solid food such as a pudding, topping, sauce, puree, cooked cereal, or salad dressing, for instance, or by otherwise adding to a food. The compound may comprise one or more inert ingredients, especially, if it is desirable to limit the amount of calories added to the diet. For example, the compounds identified by the present invention may also contain optional ingredients including, for example, herbs, other Vitamins than ascorbate, minerals, enhancers, colorants, sweeteners, flavourings, inert ingredients, and the like. Such optional ingredients may be either naturally occurring or concentrated forms.

According to a preferred embodiment, the composition envisaged by the present invention is a nutritional composition such as milk, yoghurt, curd, cheese, fermented milks, milk based fermented products, ice-creams, fermented cereal based products, milk based powders, infant formulae or pet food.

Likewise, the compound may be formulated in a pharmaceutical and/or a cosmetic composition, e.g. in tablets, such as multi-Vitamin tablets, sachets, liquid suspensions, dried oral supplement, wet oral supplement, dry tube-feeding, wet tube-feeding, creams, lotions, ointments etc.. It will be appreciated that the respective form of the pharmaceutical and/or cosmetic composition requires suitable excipients and/or carriers, which include e.g. maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof.

The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. In other embodiments, the supplement is provided as a powder or liquid suitable for adding by the consumer to a food or beverage.

All of the nutritional and the pharmaceutical/cosmetic compositions may contain, apart from the compound capable to increase the expression of Vitamin C transporters, also the Vitamin C itself.

According to another preferred embodiment, the compound included in the present composition is selected from natural-product extracts, phytochemicals, amino acids, fatty acids, cholesterol, minerals, sugars or a mixture thereof.

In general an amount of from 0.001-100, the latter of which may be represented by an oral supplement embodiment, preferably of from 0.05 to 50 wt.-% of the test-compound in the composition is envisaged, more preferably of from 0. 1 to 50, wt.-%, based on the total weight of the composition.

According to an alternative embodiment, the present compounds and/or compositions may be utilized for improving the bio-availability of Vitamin C in tissues, such as e.g. intestine, eye, liver, bone, joint, skin and brain. Since Vitamin C is highly soluble in water, effective amounts, which are taken up by cells are low in comparison to the amounts ingested. In common, the term bio-availability describes (= FDA description) the rate and extent to which the active ingredient or active moiety is absorbed from a composition product and becomes available at the site of action. The bio-availability of orally ingested drugs is determined by factors, which include the nature of the molecule, its stability, and the formulation administered - and in the patient - such as a reduced intestinal surface area as a result of colic disease or intestinal resection and whether or not the drug is taken with a meal. Factors influencing the bio-availability may include, but are not limited to a poor absorption from the gastrointestinal tract, hepatic first-pass effect and metabolisation and degradation of the drug prior to reaching system circulation. The current recommended dietary allowance (RDA) of Vitamin C concerns also its bio-availability and amounts to 60 mg/day for adults.

According to an embodiment of the present invention the composition is used for the treatment or prevention of a disease accompanied by a low reductive potential in an individual, such as coronary heart diseases, cancer, neurodegenerative diseases, and ageing. Since the skin, as the largest organ of a living being is exposed to detrimental environmental influences, the present invention particularly envisages the use of the present compounds for the treatment and/or prevention of conditions brought about by stress exerted on the skin. A stress shall be interpreted to comprise physical stress, e.g. ultraviolet irradiation of the skin, chemical stress, such as exposure to chemical agents, pollutants, or allergenic material, or biological stress, such as allergic reactions or being exposed to or infected by microorganisms, such as pathogenic bacteria, viruses, fungi etc..

In principle, such conditions comprise any kind of pathological processes, such as dermatological diseases or generally involving free radicals.

According to a preferred embodiment the present composition is used for the treatment and/or prevention of a disease or stress of a particular tissue including primary lesions which is a physical alteration of the tissue considered to be caused directly by the disease process and secondary skin lesions resulting from external factors such as itching, trauma, or changes caused by healing. For e.g. the skin, primary skin lesions may comprise atopic dermatitis, psoriasis, bacterial dermatitis and skin lesions caused by viral infections.

According to yet another embodiment the present invention also provides a method for identifying compounds having the capability to modulate the expression of Vitamin C transporters in such a way that either the expression of Vitamin C transporters is increased or decreased . The term "increase" generally designates an increased expression and the term "decrease" is directed to a lowered expression, wherein the addition of the compound to be tested leads to a detectable change in the expression rate of the Vitamin C transporters and/or in the Vitamin C content of the tissue cells.

The following examples illustrate the invention without limiting it thereto.

### Examples

### Real time PCR based in-vitro test for monitoring the modulation gene expression

Primary adult epidermal keratinocytes (NHEK) for Cambrex Company (USA, Ordering number: CC-2501) have been used in this study. They were grown in KGM medium (Cambrex Company, USA, Ordering number: CC-3101 and CC-4131) on fibronectin and collagen type I coated plastic dishes (6- or 12-well plates) at 37°C and 5% CO₂ and expanded until passage 4 where they were used for further experiments.

NHEK cells at passage 4 were seated in 6 well plates at a density of 5000 cells/cm2, determined by means of a Thoma chamber under a light microscope. Cells were grown to 100% confluence within 5-6 days with medium changed every second day and were further used for in vitro-test for gene expression as depicted schematically in Fig. 1.

After the envisaged density has been reached, the test compounds were applied (rosemary extract (0.1% (w/v) and 0.25% (w/v) as aqueous extract from different origins or hydrolyzed and non-hydrolyzed ethylacetate extracts, 10 µM gingko bilba extract, 10 µM epigallocatechingallate (EGCg), 10 µM (R,S)-equol, 10 µM quercetin, 10 µM hesperetin, 10 µM curcumin or 10 µM genistein) or the same amount of solvent used to dissolve the test compounds which served as a negative controls. Cells with applied test compound or negative control were incubated at 37°C and 5% CO₂ for an additional 4h, 8h, 16h or 24h. After removal of the supernatant the cells were rinsed twice with Dubellco's phophate buffered saline (PBS, Sigma, Germany), cells were lysed with 350 µl of a lysis buffer (Buffer RL, Qiagen AG, Basel, Switzerland) containing 1% β-mercaptoethanol (Sigma, USA) and total RNAs from cell lysates were extracted using the QIAshredder column and the RNeasy® Mini Kit (Qiagen AG, Basel, Switzerland). RNA quantification was performed using the Ribogreen® RNA quantitation Kit (Molecular Probes, USA) on 96-well plates and a fluorescence microplate reader (Spectra fluor plus F 129005, Tecan). The measurements were done in duplicate. The samples were diluted either 1:680 or 1:3400 in a final volume of 100 µl 1xTE buffer. Dilutions of the ribosomal RNA in a concentration of 1, 0.5, 0.1, 0.02, 0 µg/ml were used as standards and the measurement was performed by fluorimetry at 485 nm and 538 nm. RNA quantification was then calculated compared to the standard curve. Integrity of RNA was controlled using agarose gel electrophoresis (1% agarose in TBE: 90 mM Tris/HCl pH 8.0, 80 mM Boric acid, 3 mM EDTA in ddH₂O).

For reverse transciption 2µl pd(N)₆ random hexamers (Amersham Biosciences, Art. n°272080, England) and 1µl dNTP (10 mM, Amersham Biosciences, Art. n° 272050, -60, - 70, -80, England) were added to 2µg RNA in nuclease-free water (Ambion, USA) in a final volume of 12µl. After 5min incubation at 65°C, samples were immediately placed on ice and quickly centrifuged. Then, 4µl of 5X first strand buffer (250 mM NaCl, 0.1 mM EDTA, 1 mM DTT, 0,1 % (V/v) NP-40, 50% (v/v) glycerol), 2µl of dithiothreitol, 1µl of RNase inhibitor and 1µl of reverse transcriptase SuperScript II RNase H⁻ (SUPERase-In, 20U/µl, Ambion, Art. n°2694, USA) were added (final volume 20µl). The reverse transcription reaction was performed in a PCR cycler (PTC-100^{™}, Programmable Thermal Controller, MJ Research Inc., USA) using the following temperature program: activation of the enzyme: 10min at 25°C; reverse transcription reaction: 60 min at 42°C; inhibition of the enzyme: 20min at 70°C. The sample was then kept in the freezer at -20°C until further use.

The real-time PCR was performed according to the TaqMan^{®} method in 96 well plates (96WP) using assays-on-demand primer and probes (SVCT1: Hs00195277_ml, SVCT2:, Hs00192765_ml, 18S-rRNA: 4310893E ,GAPDH: 4310884E ,Applied Biosystems, USA). Analysis was done in triplicate using a master mix (3.5x) which contained 43.7µl TaqMan^{®} 2x Universal PCR master mix (Applied Biosystems, USA), 4.4µl assays-on-demand primers and probes, 21.9µl nuclease-free water and 17.5µl cDNA (87.5ng = 25ng per replicate). Triplicates of 25µl master mix were loaded on a 96 well ABI PRISM reaction plate (Applied Biosystems, USA), covered with a transparent optical adhesive cover and centrifuged three times at 2000rpm for 1min or until all air-bubbles had been removed. The PCR reaction was then performed in the ABI PRISM^{®} 7000 Sequence Detection System (Applied Biosystems, USA) using the following temperature program: activation of the enzyme: 2min at 50°C; denaturation: 10min at 95°C and 40 cycles target amplification: 15sec annealing at 95°C and 1min extension at 60°C. The analysis of the amplification plots was done using the ABI PRISM^{®} software Baseline adjustments were done individually (SVCT1: 17-27, SVCT2: 6-15, 18S-rRNA: 2-12; GAPDH: 8-18), whereas thresholds were set manually at 0.2 for all primers. Gene expression was normalized to either GAPDH or 18S-rRNA and statistical analysis was done by ANOVA.

The treatment with rosemary extract resulted in a significant decrease of the expression of SVCT1 expression in the real-time PCR in comparison to the negative control, whereas SVCT2 expression was not altered (cf. Fig. 2). Interestingly, the addition of said compound in different amounts (0.1% (w/v) and 0.25% (w/v) per well, at constant assay conditions) led to a dose-dependent decrease of the expression. The addition of gingko biloba extract, (-) epigallocatechin gallate (EGCg), equol, quercitin, hesperetin/ hesperidin, curcumin and genistein at the concentrations of 10 µM, however, did not show any influence towards SVCT1 and SVCT2 expression.

### ¹⁴C-labelled vitamin C based in-vitro test for monitoring the vitamin C uptake

Human immortalized keratinocytes (HaCaT) were seeded on 6-well or 12-well plastic dishes at a density of 20'000 cells/cm² and grown in Dulbecco's Modified Eagles Medium containing 4500 mg/l glucose, supplemented with penicillin and streptomycin (each 100 U/ml), 10% fetal bovine serum and 0.584 g/L glutamine until confluency at 37°C, 95% relative humidity and 5% CO₂. The medium was changed every second day.

A 50 mM stock solution of ¹⁴C-labelled ascorbic acid was prepared by dissolving 1.85MBq (Amersham Biosciences, UK) in 77µl transfer buffer (140 mM NaCl, 4.2 mM KHCO₃, 5.8 mM KCl, 1.3 mM CaCl₂, 0.5 mM MgCl₂, 10 mM Hepes, 1% Penicillin Streptomycin, 0.1 mM DTT (freshly added)) under semi-sterile conditions. This stock solution was used immediately and remaining solution was stored at -20°C for a maximal time period of 2 weeks.

Confluent HaCaT cells were incubated with or without test compound (aqueous FeCl₃ solution in different concentrations) for 18-24h and eventually stressed for further 2h with 50 µM menadione, 500 µM H₂O₂ or conditioned medium obtained from UV-irradiated HaCaTs. Then cells were washed twice with PBS and incubated with 300µl or 400µl of ¹⁴C-labelled ascorbic acid at different concentrations of (5 µM, 50 µM or 250 µM) by diluting the prepared stock solution in the appropriate amount of transfer buffer. After incubation at 37°C for different periods of time (5, 10, 30, 60, 90 and 120 min) cells were immediately placed on ice, supernatant removed and frozen, cells washed twice with ice-cold PBS and finally harvested with a cell scraper and 300µl or 400µl urea buffer (9.5M urea, 10 mM Tris/HCl pH 8.0, 2 mM EDTA pH 8.0). Amount of radiolabel in supernatant and cell lysate was then measured by scintillation counting of a dilution of 50µl supernatant or 200µl of cell lysate in 9ml scintillation cocktail. Obtained data were analysed by blotting incubation time versus ascorbic acid up-take (either as percentage or nmolar).

Other compounds were also found to increase ascorbic acid up-take in the oxidative stress model using similar experimental conditions as described above. Such compounds exhibiting said trait were glutathione, N-acetyl-cysteine and lysine (data not shown).

## Claims

1. Use of a compound capable of modulating expression of Vitamin C transporters in cells selected from natural-product extracts, phytochemicals, amino acids, fatty acids, cholesterol, minerals, sugars or a mixture thereof, for the preparation of a nutritional or pharmaceutical composition for modulating the bio-availability of Vitamin C in tissues.

2. Use of a compound according to claim 1 for the treatment or prevention of a disease or stress in a tissue of interest, preferably in the intestine, eye, liver, bone, joints, skin and the brain.

3. The use according to claim 2, wherein the disease or stress of the skin is dermatitis, psoriasis, bacterial dermatitis and skin lesions caused by viral infections or scratching, trauma, or changes caused by healing.

4. The use according to claim 1, wherein said compound is capable of modulating expression by increasing expression of Vitamin C transporters.

5. The use of a compound according to claim 4, wherein said compound is selected from iron or a salt thereof, glutathione, N-acetyl-cysteine and lysine.

6. The use according to claim 1, wherein said compound is capable of modulating expression by decreasing expression of Vitamin C transporters.

7. The use of a compound according to claim 6, wherein said compound is selected from the group consisting of rosemary extract, sulforaphane, all-trans retinoic acid and zinc or a salt thereof.

8. The use according to claim 1 to 7, wherein Vitamin C is added to said compound.
